(19) Europäisches Patentamt
European Patent Office
Office européen des brevets

(11) **EP 3 490 612 B1**

(12) **EUROPEAN PATENT SPECIFICATION**

(45) Date of publication and mention
of the grant of the patent:
**21.08.2024 Bulletin 2024/34**

(21) Application number: **17833597.2**

(22) Date of filing: **28.07.2017**

(51) International Patent Classification (IPC):
**G01N 33/574** (2006.01)

(52) Cooperative Patent Classification (CPC):
**C12Q 1/6886; G01N 33/48; G01N 33/57419;
G01N 33/57442;** C12Q 2600/154; G01N 2440/12;
G01N 2800/60

(86) International application number:
**PCT/CN2017/094917**

(87) International publication number:
**WO 2018/019294 (01.02.2018 Gazette 2018/05)**

(54) **METHODS FOR GYNECOLOGIC NEOPLASM DIAGNOSIS**

VERFAHREN ZUR DIAGNOSE VON GYNÄKOLOGISCHEN NEOPLASMEN

MÉTHODES DE DIAGNOSTIC DE NÉOPLASME GYNÉCOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.07.2016 US 201662368182 P**

(43) Date of publication of application:
**05.06.2019 Bulletin 2019/23**

(73) Proprietor: **Guzip Biomarkers Corporation
Taiwan 23557 (TW)**

(72) Inventor: **LAI, Hung-Cheng
Taipei City 114 (TW)**

(74) Representative: **Hoefer & Partner Patentanwälte
mbB
Pilgersheimer Straße 20
81543 München (DE)**

(56) References cited:
**WO-A1-2005/115388     WO-A1-2016/115354
WO-A2-2012/120374     CN-A- 101 230 399**

- **RUI-LAN HUANG ET AL: "Integrated Epigenomics Analysis Reveals a DNA Methylation Panel for Endometrial Cancer Detection Using Cervical Scrapings", CLINICAL CANCER RESEARCH, vol. 23, no. 1, 9 August 2016 (2016-08-09), US, pages 263 - 272, XP055446410, ISSN: 1078-0432, DOI: 10.1158/1078-0432.CCR-16-0863**
- **CHOI: "High-throughput DNA hypermethylation profiling in different ovarian epithelial cancer subtypes using universal bead array", ONCOLOGY REPORTS, 2 September 2010 (2010-09-02), XP055267272, ISSN: 1021-335X, DOI: 10.3892/or_00000937**
- **CHEN, Y.C. ET AL.: "Quantitative DNA methylation analysis of selected genes in endometrial carcinogenesis", TAIWAN ESE JOURNAL OF OBSTETRICS & GYNECOLOGY, vol. 54, no. 5, 31 December 2015 (2015-12-31), pages 572 - 579, XP055282084**
- **YOON, M.S. ET AL.: "High-throughput DNA hypermethylation profiling in different ovarian epithelial cancer subtypes using universal bead array", ONCOLOGY REPORTS, 31 December 2010 (2010-12-31), pages 917 - 925, XP055267272**
- **HUANG, R.L. ET AL.: "Integrated epigenomics analysis reveals a DNA methylation panel for endometrial cancer detection using cervical scrapings", CLINICAL CANCER RESEARCH, vol. 23, no. 1, 9 August 2016 (2016-08-09), pages 1 - 35, XP055446410**

- **LEE, J. ET AL.: "Presence of 5-methylcytosine in CpNpG trinucleotides in the human genome", GENOMICS, vol. 96, no. 2, 9 April 2010 (2010-04-09), pages 67 - 72, XP027151917**

Remarks:
    The complete document including Reference Table(s) and the Sequence Listing(s) can be downloaded from the EPO website

**Description**

**BACKGROUND OF THE INVENTION**

1. FIELD OF THE INVENTION

The present disclosure relates to cancer diagnosis. More particularly, the disclosed invention relates to method for cancer diagnosis based on the methylation state of selected markers.

2. DESCRIPTION OF RELATED ART

Gynecologic cancer is the uncontrolled growth of abnormal cells that originate from women's reproductive organs. Main types of gynecologic cancers include cervical, ovarian, endometrial/uterine (hereinafter, endometrial), vaginal, and vulvar cancers, while some rare gynecologic cancers include gestational trophoblastic disease (GTD) and primary peritoneal cancer.

Endometrial cancer (EC) is the leading cause of gynecologic cancer in the Sates, and ranks as the sixth-most diagnosed female cancer worldwide. According to the GLOBOCAN project of the World Health Organization, in 2012, the incidence and mortality of EC were about 310,000 and 76,000, respectively, and it estimated that more than 380,000 new cases and 93,000 deaths from EC are expected in 2020

Most endometrial cancers are found in women who are going through or have gone through menopause. However, for those who do not have any signs or symptoms, there are no simple and reliable ways to test for uterine cancer. For patients showing symptoms or at a higher risk for endometrial cancer, an endometrial biopsy or a transvaginal ultrasound may be performed to help diagnose or rule out uterine cancer.

EC may be broadly classified into two types by histopathological assessment. Type I ECs comprise well to moderately differentiated (grades 1 to 2) endometrioid adenocarcinomas. Type II ECs comprise serous type, clear cell, and poorly differentiated (grade 3) endometrioid-type forms. Most type I ECs are diagnosed at an early stage (stage I or II) and have a favorable outcome, whereas type II ECs are typically diagnosed at an advanced stage and have a relatively poor prognosis. Although most patients with an endometrioid-type EC are diagnosed at the early stage, 15% of patients with a late-stage endometrioid EC have a poor 5-year survival rate of less than 50% (40-50% for stage III, 15-20% for stage IV). Early detection remains the best way to improve outcome. However, there are currently no satisfactory methods for EC screening.

Abnormal uterine bleeding is the most frequent symptom of EC, but many other disorders give rise to the same symptom. Even when bleeding occurs in postmenopausal women, only 10% of cases are caused by the EC. The choice of the ideal detection strategy depends upon the sensitivity, specificity, probability of accuracy, and cost. Transvaginal ultrasound (TVU) is used to exclude EC. The cutoff value for TVU in symptomatic premenopausal women and those taking hormone-replacement therapy, however, is lower because of variations in the endometrial thickness under the influence of circulating female steroid hormones. Endometrial samples obtained by suction curettage in an outpatient setting may have a higher sensitivity and specificity compared with TVU. Yet, the failure rate of this invasive procedure can be up to 54%. The clinical guidelines recommend fractional dilatation and curettage (D & C) under anesthesia if the endometrial sampling is inadequate or inclusive for diagnosis. Thus, many patients undergo repeated invasive evaluations by endometrial sampling or D & C, which is inconvenient, stressful, and costly.

On the other hand, the diagnostic accuracy of the hysteroscopy can achieve an overall sensitivity of 86.4% and specificity of 99.2% in both pre- and post-menopausal women. Nonetheless, there is debate over the best cutoff value for endometrial thickness diagnosed with TVU that should warrant endometrial sampling or hysteroscopy. Noninvasive methods to detect the presence or absence of EC are measurement of the serum and cervicovaginal concentrations of cancer antigen 125 (CA-125). The accuracy of using CA-125 as an indicator is easily confounded by benign conditions such as adenomyosis or uterine myomas and endometriosis. Cytology from cervical scrapings have also been used for detecting ECs, but the rate of abnormal results ranges from 32.3% to 86.0% for type I EC and 57.1% to 100% for type II EC.

In view of the foregoing, there exists a need in the related art for providing methods for use in the diagnosis of gynecologic tumor, in particular, endometrial cancer. CHEN, Y.C. et al. (TAIWANESE JOURNAL OF OBSTETRICS & GYNECOLOGY, vol. 54, no. 5, 31 December 2015 (2015-12-31), pages 572-579) and YOON, M.S. et al. (ONCOLOGY REPORTS, 31 December 2010, pages 917-925) disclose methylation markers for endometrial cancer.

**SUMMARY**

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

The present invention is directed to a method for assessing whether a subject has an endometrial cancer (EC) according to claim 1.

According to one embodiment of the present disclosure, the method comprises the following steps:

(a) obtaining a sample from the subject;
(b) determining the methylation state of at least one target gene in the sample, wherein the at least one target gene is BHLHE22 (SEQ ID NO. 1) or THY1 (SEQ ID NO. 2) or both;
(c) determining whether the at least one target gene is hypermethylated; and
(d) assessing whether the subject has the EC based on the result of the step (c), wherein the hypermethylation of the at least one target gene indicates that the subject has the EC.

According to various embodiments of the present disclosure, the sample is a sample obtained from a subject, preferably a human subject, or present within a subject, preferably a human subject. For example, the sample is obtained from the endometrial tissue or cell samples (e.g., cervical scraping cells) of the subject.

According to some embodiments of the present disclosure, the at least one target gene further comprises at least one of the following additional genes: ADARB2 (SEQ ID NO. 3), CDO1 (SEQ ID NO. 4), CELF4 (SEQ ID NO. 5), CLVS2 (SEQ ID NO. 6), GATA4 (SEQ ID NO. 7), HOXA9 (SEQ ID NO. 8), MTMR7 (SEQ ID NO. 9), NTM (SEQ ID NO. 10), PRKCDBP (SEQ ID NO. 11), TBX5 (SEQ ID NO. 12), and ZNF662 (SEQ ID NO. 13). In these embodiments, the methylation state of BHLHE22 and/or THY1, and the methylation state of at least one of the additional genes are determined, and in the step (d) the assessment is made based on the hypermethylation states of said selected genes.

According to other embodiments, the at least one target gene comprises BHLHE22, CDO1, THY1, CELF4, CLVS2, GATA4, NTM, PRKCDBP, and TBX5.

According to certain embodiments, the at least one target gene comprises BHLHE22 and CDO1. In other cases, the at least one target gene comprises BHLHE22 and CELF4. Alternatively, the at least one gene comprises BHLHE22, CDO1, and CELF. In these cases, in the step (b), the methylation states of BHLHE22 and one or both of CDO1 and CELF4 are determined, and in the step (d) the assessment is made based on the hypermethylation states of BHLHE22 and one or both of CDO1 and CELF4. In optional embodiments of the present disclosure, the sample is obtained from cervical scraping cells of the subject.

According to various embodiments of the present disclosure, the endometrial cancer is type I endometrial cancer, whereas in other embodiments, the endometrial cancer is type II endometrial cancer.

Optionally, the step of determining the methylation state of a gene, as described in the above-mentioned embodiments of the present disclosure, can be achieved by performing methylation-specific polymerase chain reaction (MSP), quantitative methylation-specific polymerase chain reaction (qMSP), bisulfite sequencing (BS), bisulfite pyrosequencing, microarrays, mass spectrometry, denaturing high-performance liquid chromatography (DHPLC), pyrosequencing, methylated DNA immunoprecipitation (MeDIP or mDIP) coupled with quantitative polymerase chain reaction, methylated DNA immunoprecipitation sequencing (MeDIP-seq), or nanopore sequencing.

In another aspect, the present invention is directed to a method for assessing whether a subject has a gynecologic carcinoma or colon carcinoma.

According to one embodiment of the present disclosure, the method comprises the following steps:

(a) obtaining a sample from the subject;
(b) determining the methylation state of at least one target gene in the sample, wherein the at least one target gene is selected from the group consisting of, ADARB2 (SEQ ID NO. 3), CLVS2 (SEQ ID NO. 6), HOXA9 (SEQ ID NO. 8), MTMR7 (SEQ ID NO. 9), and NTM (SEQ ID NO. 10);
(c) determining whether the at least one target gene is hypermethylated; and
(d) assessing whether the subject has the gynecologic carcinoma or colon carcinoma based on the result of the step (c), wherein the hypermethylation of the at least one target gene indicates that the subject has the gynecologic carcinoma or colon carcinoma.

According to various embodiments of the present disclosure, the sample is a sample obtained from a subject, preferably a human subject, or present within a subject, preferably a human subject. For example, the sample is obtained from the endometrial tissue or cell samples (e.g., cervical scraping cells) of the subject.

In some embodiments of the present disclosure, the gynecologic carcinoma is type I endometrial carcinoma, type II endometrial carcinoma, ovarian carcinoma, vaginal carcinoma, vulvar carcinoma, gestational trophoblastic disease (GTD) and primary peritoneal carcinoma.

Optionally, the step of determining the methylation state of a gene, as described in the above-mentioned embodiments of the present disclosure, can be achieved by performing methylation-specific polymerase chain reaction (MSP), quantitative methylation-specific polymerase chain reaction (qMSP), bisulfite sequencing (BS), bisulfite pyrosequencing, microarrays, mass spectrometry, denaturing high-performance liquid chromatography (DHPLC), pyrosequencing, methylated DNA immunoprecipitation (MeDIP or mDIP) coupled with quantitative polymerase chain reaction, methylated DNA immunoprecipitation sequencing (MeDIP-seq), or nanopore sequencing.

In still another aspect, the present disclosure is directed to the use of biomarker(s) identified by the present disclosure. In particular, the biomarker(s) may be used to prepare a diagnostic kit for assessing whether a subject has the carcinoma associated with one or more of these biomarkers.

As could be appreciated, diagnostic kits comprising materials suitable for measuring the methylation status of said biomarker(s) are also included in the scope of the present disclosure.

Many of the attendant features and advantages of the present disclosure will becomes better understood with reference to the following detailed description considered in connection with the accompanying drawings.

## BRIEF DESCRIPTION OF THE DRAWINGS

The present description will be better understood from the following detailed description read considering the accompanying drawings, where:

Figure 1 presents a correlation matrix (panel A) and heatmap (panel B) demonstrating the methylation signatures of candidate genes in tissues of public data from our MBDcap-Seq and Cancer Genome Atlas (TCGA), according to one working example of the present disclosure;

Figure 2 provide dot plots showing the distribution of methylation status for BHLHE22, CDO1, CELF4, and ZNF662 (panel A), and the performance of said four genes (panel B), according to one working example of the present disclosure;

Figure 3 summarizes the DNA methylation level of 15 genes using individual endometrial tissue for endometrial cancer detection, according to one working example of the present disclosure;

Figure 4 summarizes the DNA methylation level of 1 genes using individual cervical scrapings for endometrial cancer detection, according to one working example of the present disclosure;

Figure 5 provides plots indicating the performance of candidate genes using the sample from the training set (panel A) and testing set (panel B), according to one working example of the present disclosure; and

Figure 6 summarizes the results of cross-testing of six candidate genes in other colon, cervix, or ovarian carcinoma, according to one working example of the present disclosure.

## DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

For convenience, certain terms employed in the specification, examples and appended claims are collected here. Unless defined otherwise, all technical and scientific terms used herein have the same meaning as commonly understood by one of the ordinary skill in the art to which this invention belongs.

Unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more. Furthermore, the phrases "at least one of A, B, and C", "at least one of A, B, or C" and "at least one of A, B and/or C," as use throughout this specification and the appended claims, are intended to cover A alone, B alone, C alone, A and B together, B and C together, A and C together, as well as A, B, and C together.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques. Ranges can be expressed herein as from one endpoint to another endpoint or between two endpoints. All ranges disclosed herein are inclusive of the endpoints, unless specified otherwise.

As used herein, the term "diagnosis" refers to the identification of a pathological state, disease, or condition, such as neoplasms of various gynecologic tissue origins, including, cervix, ovary, endometrium/uterus, vagina, vulvar, uterus, and peritoneum lining the uterus. In some cases, the term diagnosis also refers to distinguishing between the malignant and benign neoplasms. In some other cases, the term diagnosis refers to distinguishing between the malignant neoplasm and normal tissues.

Throughout the present disclosure, the term "neoplasm" refers to a new and abnormal growth of cells or a growth of abnormal cells that reproduce faster than normal. A neoplasm creates an unstructured mass (a tumor), which can be either benign or malignant. The term "benign" refers to a neoplasm or tumor that is noncancerous, e.g. its cells do not invade surrounding tissues or metastasize to distant sites; whereas the term "malignant" refers to a neoplasm or tumor that is metastatic, invades contiguous tissue or no longer under normal cellular growth control.

As used herein, the term "cancer" refers to all types of cancer, or malignant neoplasm or tumor found in animals. The methods according to various embodiments of the present disclosure are directed to the diagnosis one or more carcinoma of gynecologic origin. The term "carcinoma" refers to a malignant tumor originating from epithelial cells. Exemplary gynecologic carcinomas of embodiments of the present disclosure include, but are not limited to, ovarian cancer, endometrial cancer, vaginal cancer, vulvar cancer, and primary peritoneal cancer.

The term "methylation" as used herein, refers to the covalent attachment of a methyl group at the C5-position of cytosine within the CpG dinucleotides of the core promoter region of a gene. The term "methylation state" refers to the presence or absence of 5-methyl- cytosine (5-mCyt) at one or a plurality of CpG dinucleotides within a gene or nucleic acid sequence of interest. As used herein, the term "methylation level" refers to the amount of methylation in one or more copies of a gene or nucleic acid sequence of interest. The methylation level may be calculated as an absolute measure of methylation within the gene or nucleic acid sequence of interest. Also, a "relative methylation level" may be determined as the amount of methylated DNA, relative to the total amount DNA present or as the number of methylated copies of a gene or nucleic acid sequence of interest, relative to the total number of copies of the gene or nucleic acid sequence. Additionally, the "methylation level" can be determined as the percentage of methylated CpG sites within the DNA stretch of interest.

As used herein, the term "methylation profile" refers to a set of data representing the methylation level of one or more target genes in a sample of interest. In some embodiments, the methylation profile is compared to a reference methylation profile derived from a known type of sample (e.g., cancerous or noncancerous samples or samples from different stages of cancer).

As used herein, the term "differential methylation" refers to a difference in the methylation level of one or more target genes in one sample or group, as compared with the methylation level of said one or more target genes in another sample or group. The differential methylation can be classified as an increased methylation ("hypermethylation") or a decreased methylation ("hypomethylation"). As used herein, the term "hypermethylation" of a target gene in a test sample refers to an increased methylation level of at least 10%, relative to the average methylation level of the target gene in a reference sample. According to various embodiments of the present disclosure, the increased methylation level may be at least 15, 20, 25, 30, 35, 40, 45, or 50%.

As could be appreciated, all the genes or polynucleotide sequences described herein respectively comprise their variants that have at least 75% nucleotide sequence identity to the named genes or polynucleotide sequences. Further, the target gene sequences also encompass the bisulfite conversion nucleotide sequence thereof. Accordingly, unless otherwise expressly specified, all of the genes or polynucleotide sequences described herein should be understood as modified in all instances by the phrase "and a bisulfite conversion nucleotide sequence thereof, and a polynucleotide sequence having at least 75% nucleotide sequence identity to said gene or said bisulfite conversion nucleotide sequence."

"Percentage (%) nucleotide sequence identity" with respect to a gene or nucleotide sequence identified herein is defined as the percentage of nucleotide residues in a candidate sequence that are identical with the nucleotide residues in the referenced polynucleotide sequence, after aligning the sequences and introducing gaps, if necessary, to achieve the maximum percent sequence identity, and not considering any conservative substitutions as part of the sequence identity. Alignment for purposes of determining percentage sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. The percentage nucleotide sequence identity of a given polynucleotide sequence A to a referenced polynucleotide sequence B (which can alternatively be phrased as a given polynucleotide sequence A that has a certain % nucleotide sequence identity to a referenced polynucleotide sequence B) is calculated by the formula as follows:

$$\frac{X}{Y} \times 100\%$$

where X is the number of nucleotide residues scored as identical matches by the sequence alignment program BLAST in that program's alignment of A and B, and where Y is the total number of nucleotide residues in A or B, whichever is shorter.

The terms "subject" and "patient" are used interchangeably herein and are intended to mean an animal including the human species that can be subjected to the diagnosis methods of the present invention. Accordingly, the term "subject" or "patient" comprises any mammal, which may benefit from the method of the present disclosure. The term "mammal" refers to all members of the class Mammalia, including humans, primates, domestic and farm animals, such as rabbit, pig, sheep, and cattle; as well as zoo, sports or pet animals; and rodents, such as mouse and rat. The term "non-human mammal" refers to all members of the class Mammalis except human. In one exemplary embodiment, the patient is a human.

The term "sample" used herein comprises any samples obtained from a patient. According to embodiments of the present disclosure, the sample contains DNA molecules and the methylation level thereof can be determined. Examples of such body samples include, but are not limited to, blood, smears, sputum, urine, stool, liquor, bile, gastrointestinal secretions, lymph fluid, osteosarcoma marrow, organ aspirates and organ or tissue biopsies. These body samples can be obtained from the patient by routine measures known to persons having ordinary skill in the art. Further, persons having ordinary skill in the art are also familiar with methods and reagents for the DNA isolation from the sample, e.g. extraction with phenol/chloroform or by means of commercial kits.

The present disclosure is based, at least in part, on the finding that differential methylation (in particular, hypermethylation) of one or more target genes, as identified hereinbelow, relates to the tumor progression, or the absence thereof, in a subject. Accordingly, these target genes, alone or in combination, can be used as biomarkers for the prediction of risk or susceptibility of a subject developing a neoplasm, the determination of the malignancy of the neoplasm. Further, the methylation profile of relevant genes of the patient can be used as a guide for tailoring suitable therapy regime individually. For example, for patients with one or more hypermethylated target genes listed herein, de-methylation agents or other epigenetic drugs can be administered to the patients to treat the neoplasm.

In view of the foregoing, the present disclosure provides various diagnostic methods, which will be separately addressed below. For example, all methods involve the determination of the methylation state or methylation level of at least one target gene. Hence, the steps common to most, if not all, claimed methods are first described in the following paragraph.

According to various aspects and/or embodiments of the present disclosure, common steps for the methods of the present disclosure include, at least, (a) a sample retrieval step, (b) at least one methylation state determination step; and (c) at least one hypermethylation determination step.

In the sample retrieval step, for methods that involves a live subject at least, a biological sample is obtained from the subject. According to various embodiments of the present disclosure, the sample is a sample obtained from a subject, preferably a human subject, or present within a subject, preferably a human subject, including a tissue, tissue sample, or cell sample (e.g., a tissue biopsy, for example, an aspiration biopsy, a brush biopsy, a surface biopsy, a needle biopsy, a punch biopsy, an excision biopsy, an open biopsy, an incision biopsy an endoscopic biopsy, cervical scraping cells, uterus scraping cells or a vaginal lavage), tumor, tumor sample, or biological fluid (e.g., peritoneal fluid, blood (including plasma), serum, lymph, spinal fluid). According to certain working examples of the present disclosure, the sample is obtained from the ovarian tissue, cell samples (e.g., cervical scraping cells) and body fluid (e.g., serum and plasma) of the subject. As to methods that are directly practiced on existing biological samples, such as tissue biopsy samples, cervical scraping cells, and body fluid samples (e.g., blood or urine), the afore-mentioned sampling step is omitted and the existing sample is retrieved for subsequent analysis.

Regarding the step (b), the methylation state is determined using qMSP or bisulfite pyrosequencing, according to working examples provided below. However, as could be appreciated, the present method is not limited to the methods described above; rather, the scope of the claimed invention encompasses the use of other equivalent methods for quantitatively determining the methylation state or level of a particular gene. As could be appreciated, the above-mentioned methods and equivalents thereof are also applicable to the embodiments described hereinbelow, hence, the method suitable for determining the methylation state or methylation level of the gene is not repeated in the following aspects/embodiments, for the sake of brevity.

In the hypermethylation determination step, the presence or absence of hypermethylation of said at least one target gene is determined.

In the first aspect, the present disclosure provides a method for assessing whether a subject has an endometrial cancer (EC), which comprises the sample retrieval step, the methylation determination step; and the assessment step, as described above, and an assessment step (d), in which the presence or absence of the EC in the subject is determined based on the result of the step (c). Specifically, the hypermethylation of the at least one target gene indicates that the subject has the EC. According to some embodiments of the present disclosure, the absence of the hypermethylation of the at least one target gene indicates that the subject does not have the EC. According to some embodiments of the present disclosure, the absence of the hypermethylation of the at least one target gene indicates that the subject does not have an endometrial neoplasm or the endometrial neoplasm is benign; for example, myoma is a common benign endometrial neoplasm.

According to various embodiments of the present disclosure, the at least one target gene is BHLHE22 or THY1 or both.

In to some embodiments, in addition to BHLHE22 and/or THY1, the target gene further comprises at least one of ADARB2, CDO1, CELF4, CLVS2, GATA4, HOXA9, MTMR7, NTM, PRKCDBP, TBX5, and ZNF662.

According to certain optional embodiments, the at least one target gene comprises BHLHE22 and CDO1. In some embodiments, the at least one target gene comprises BHLHE22, CDO1, and TBX5. In some other embodiments, the at least one target gene comprises BHLHE22, THY1, CDO1, and CLVS2. In still some other embodiments, the at least one target gene comprises BHLHE22, CDO1, and CELF4. As another example, the at least one target gene comprises BHLHE22 and CELF4. Alternatively, the at least one target gene comprises THY1 and CDO1. Still alternatively, the at least one target gene comprises THY1 and CDO1, as well as one or both of CLVS2 and GATA4. In some embodiments, the at least one target gene comprises BHLHE22, CDO1, THY1, CELF4, CLVS2, GATA4, NTM, PRKCDBP, and TBX5.

In the embodiments where the methylation state of more than one target gene is determined, the assessment may be made based on the presence or absence of the hypermethylation of said target genes.

According to various embodiments of the present disclosure, the sample is derived from the cervical scraping cells or endometrial sample of the subject.

According to certain embodiments of the present disclosure, the present method is used for assessing whether the subject has endometrial carcinoma at an early stage (stage I or II according to International Federation of Gynecological and Obstetrics principle). In some optional embodiments, the sample is derived from the cervical scraping cells of the subject, and the hypermethylation of BHLHE22 and at least one of CDO1 and CELF4 is an indication that the subject has the endometrial carcinoma. According to various embodiments of the present disclosure, the endometrial carcinoma may be type I or type II endometrial carcinoma. As compared to conventional diagnostic tools in which the type II endometrial carcinoma is often diagnosed in a later stage of cancer development (e.g., stage 3 or 4), the present method is of particular advantages for providing a diagnosis before the cancer advances to a more malignant stage.

As could be appreciated, the biomarkers identified in the present disclosure could be used to prepare diagnostic tools for assessing whether a subject has the endometrial carcinoma.

For example, the kit may comprise materials that could be used to determining the methylation level of one or more of the above-identified biomarkers, such as, BHLHE22, THY1, ADARB2, CDO1, CELF4, CLVS2, GATA4, HOXA9, MTMR7, NTM, PRKCDBP, TBX5, and ZNF662.

In the second aspect, the present disclosure provides a method for assessing whether the subject has a gynecologic carcinoma or colon carcinoma. The method also comprises the common steps (a), (b) and (c) as described above, and an assessement step (d) in which the presence of the gynecologic carcinoma or colon carcinoma in the subject is determined based on the result of the step (c). In particular, the hypermethylation of the at least one target gene indicates that the subject has the gynecologic carcinoma or colon carcinoma. According to some embodiments of the present disclosure, the absence of the hypermethylation of the at least one target gene indicates that the subject does not have a gynecologic neoplasm or colon neoplasm or the gynecologic neoplasm or colon neoplasm is benign.

According to various embodiments of the present disclosure, the at least one target gene is selected from the group consisting of, ADARB2, CLVS2, HOXA9, MTMR7, and NTM.

According to certain embodiments of the present disclosure, the gynecologic carcinoma is type I endometrial carcinoma, type II endometrial carcinoma, ovarian carcinoma, vaginal carcinoma, vulvar carcinoma, gestational trophoblastic disease (GTD) and primary peritoneal carcinoma.

Similarly, the biomarkers identified in the present disclosure could be used to prepare diagnostic tools for assessing whether a subject has the gynecologic carcinoma or colon carcinoma.

For example, the kit may comprise materials that could be used to determining the methylation level of one or more of the above-identified biomarkers, such as, ADARB2, CLVS2, HOXA9, MTMR7, and NTM.

The following Examples are provided to elucidate certain aspects of the present invention and to aid those of skilled in the art in practicing this invention. These Examples are in no way to be considered to limit the scope of the invention in any manner. Without further elaboration, it is believed that one skilled in the art can, based on the description herein, utilize the present invention fully.

**Materials and Methods**

1. Tissue Specimens

All tissue specimens were collected between October 2014 and May 2016 at Taipei Medical University-Shuang Ho Hospital, New Taipei, Taiwan. The criteria for inclusion stipulated that the study participants needed to be women aged 30-80 years. Age, histological type of tumor, the International Federation of Gynecology and Obstetrics stage and histological grade were reviewed from the hospital records for each participant. Studies were conducted with approval from the Institutional Review Board of the Taipei Medical University-Shuang Ho Hospital in accordance with the Declaration of Helsinki 2000. Informed consent was obtained from all participants in this study.

The endometrial specimens were obtained during surgery and were frozen immediately in liquid nitrogen and stored at -80°C until analysis. The pathological diagnosis of each sample was confirmed with histological examination by gynecologic pathologists. Normal epithelial tissue was collected from myoma patients.

Cervical scraping cells were collected from patients diagnosed with myoma or endometrial carcinoma, and female patients were free of ovarian and cervical diseases at the sampling time. The endocervical scraping cells were collected as follows. Inserting a new endocervical brush into the endocervical canal and gently rotating the brush three to five times to ensure appropriate sampling. The brush was then plunged into a centrifuge tube (15 ml) containing 2 ml RNA*later*® (Ambion, Life technologies, USA), and closed the cap of the centrifuge tube. The endocervical scraping cells specimens were stored at 4°C. After vortexing for 10 seconds, the swab cells-containing sample was aliquoted into three microcentrifuge tubes (1 ml per tube), which were stored at -80°C until further analysis.

2. Discovery and Validation

In the discovery cohort, public data from The Cancer Genome Atlas (TCGA) data (including using 270 malignant endometrial tumor tissues and 28 normal endometrial tissues), Em-MethylCap-seq (including 78 malignant endometrial tumor tissues and 11 normal endometrial tissues), and HCL-data (including 10 malignant endometrial tumor tissues and 10 normal endometrial tissues in myoma) were used to identify the methylation biomarkers. The samples used to verify the methylation biomarkers included 25 endometrial malignant tumor tissues, 5 normal endometrium of myoma, and 15, 10, and 15 cervical scrapings collected from subjects with endometrial malignant tumor tissues, myoma and normal endometrium, respectively.

For the training set used in the validation cohort, cervical scrapings samples, each of size 28, collected from subjects with endometrial cancer, myoma, and health subjects were used. As to the testing set for the validation cohort in Example 1, samples of the same size were used. For testing set used in the validation cohort in Example 2, 50, 40, and 56 cervical scraping cell samples respectively collected from subjects with endometrial malignant tumor tissues, myoma and normal endometrium were uses.

3. Genome-wild DNA methylomics analysis

3.1. 450K Methylation Bead Array

The first methylomics analysis of 298 endometrial tissues (endometrioid-type ECs and 28 adjacent normal tissues) was performed using a Methylation 450K BeadChip array (Illumina, Inc., San Diego, CA, USA), so as to identify the differential methylation profile of EC. The level 3 data from the TCGA data portal was used. The methylation score for each CpG was represented as a $\beta$-value and normalized. The cancer carcinoma samples exhibited $\geq$40% neoplastic cellularity. We removed the probes mapped at sex chromosome and covered single nucleotide polymorphism (SNP), and focused on the remaining 379,054 CpG sites were analyzed in this study. The cancer carcinoma samples exhibited $\geq$40% neoplastic cellularity. The significantly different methylated CpG sites were analyzed using the Mann - Whitney U test and P $\leq$ 0.001. We further focused on CpG sites located closest ($\pm$1000 bp) to the transcriptional start sites (TSS) of the coding genes. Finally, we selected hypermethylated candidates with $\geq$5 remaining CpG sites in the coding gene. Also, the top 5% hypermethylated differences from the median reads of EC values minus the normal value was selected.

3.2. MethylCap Sequencing

Methylated DNA was enriched from 2 $\mu$g genomic DNA using the MethylMiner methylated DNA Enrichment Kit (Invitrogen, Carlsbad, CA) following the manufacturer's instructions. Briefly, genomic DNA was sonicated to about 200-bp, captured by MBD proteins and precipitated using 1M salt buffer. Enriched methylated DNA was used to generate libraries for sequencing following the standard protocols from Illumina (San Diego, CA). MethylCap-seq libraries were sequenced using the Illumina Genome Analyzer IIx System. Image analysis and base calling were performed using the standard Illumina pipeline. Unique reads (up to 36-bp reads) were mapped to the human reference genome (hg18) using the ELAND algorithm, with up to two base-pair mismatches.

The uniquely mapped reads were used for additional linear normalization and differential methylation analysis. The methylation level was calculated by accumulating the number of reads using the following Equations 1 and 2.

$$N_{Read,i} = \frac{U_{Read,i}}{N_U/10^{\wedge}(INT(log_{10} N_U))}$$ (Equation 1)

N_(Read,i) is the number of normalized reads at the ith bin; U_(Read,i) is the number of uniquely mapped reads at the ith bin, and N_U is the number of total uniquely mapped reads. "INT" rounds the element to the nearest integers towards minus infinity, "^" means the power operator. A region of methylation level is represented by the average of the normalized of uniquely reads. Comparison of group A and B (G = A or B), the average methylation level (AVGR,G ) was calculated separately at two groups in a given region R (which includes m bin size, and start at the sth bin). The number of sample is S_A for group A, and S_B for group B.

$$AVG_{R,G} = \frac{\sum_G M_{R,G}}{S_G} , R = (b_{s+0}, b_{s+1}, ..., b_{s+m})$$ (Equation 2)

$AVG_{R,G}$ means the average methylation level of group G at the R region. $M_{R,G}$ is the methylation levels of each sample of group G at the R region. The statistical significance of different methylation at the R region was identified using two-tailed and Mann-Whitney U test ($P \leq 0.001$). Differentially methylated loci were analyzed between case and control samples. The methylation level for a 2000 bp region spanning 1000 bp upstream and downstream of the TSS of the coding genes was analyzed. We excluded the TSS regions in the sex chromosome, and the remaining 23,215 TSS regions were analyzed in this study, in which the top 5% hypermethylated differences from the median reads of EC values minus the normal value was selected.

TCGA data portal used 450K methylation Chip array to analyze the tissue samples, and the data portal contained datasets from 417 endometrial tissues. Among them, 371 cancer cases with $\geq$ 40% neoplastic cellularity were chosen for this study. Non-parametric test was used to investigate the differential methylated CpG sites between case and control groups. Probes with $P \leq 0.001$ and the top 5% differential methylation of high $\beta$-values were initially selected. Then, significant probes located within the region spanning -1000 to +1000 relative to the transcription start site of coding genes were identified. Hypermethylated genes with at least five of the identified probes were selected per our selection criteria.

3.3. Clustering analysis of DNA methylation profile and selection of candidates

DNA methylation profiles was obtained using complete-linkage method of the agglomerative Hierarchical clustering under Manhattan distance in function of MeV4 version 4.9. In each group, one to two candidates that were less reported in methylation changes of cancers and low signals of normal endometrium in non-EC participants were selected.

4. Quantitative DNA Methylation Polymerase Chain Reaction

4.1. Preparation of Genomic DNA and Bisulfite Conversion

Genomic DNA was extracted from specimens using QIAmp DNA Mini Kit (QIAGEN GmbH, Hilden, Germany). The genomic DNA (1 $\mu$g) was bisulfite modified using the EZ DNA Methylation Kit (ZYMO RESEARCH, CA, USA), according to the manufacturer's recommendations, and dissolved in 70 $\mu$L of nuclease-free water. The bisulfite DNA was used as the temple for the DNA methylation analysis.

4.2. qMSP

Quantitative methylation-specific PCR (qMSP) using specific probes and primers was performed to identify the relative DNA methylation level by making reference to the un-methylated (COL2A1) total input DNA.

PCR products were amplified with the LightCycler 480 SYBR Green I Master (Roche, Indianapolis, IN, USA) and performed using LightCycler 480. A 20 $\mu$l reaction mixture contained 2 $\mu$L bisulfite-converted DNA, 250 nM each primer, and 10 $\mu$l Master Mix. The reactions were conducted on LightCycler 480 under the following thermal profiles:

95°C for 5 minutes, 50 cycles of 95°C for 10 seconds, 60°C for 30 seconds, and 72°C for 30 seconds, and a final extension at 72°C for 5 minutes. Duplicate testing was performed to each gene in all specimens. The DNA methylation level will estimate the $\triangle$Cp using the formula: (Cp of Gene) - (Cp of COL2A)]. Test results with Cp-values of COL2A >36 were defined as detection failure. The primers were designed by Oligo 7.0 Primer Analysis software.

5 Statistics

The Mann-Whitney nonparametric U test was used to identify differences in methylation level between two categories, and the Kruskal-Wallis test was used to identify differences in methylation level between more than two categories.

In an independence cohort, the true positive rate (i.e., the sensitivity) was plotted against the false positive rate (that is, 1-specificity) to obtain a receiver operating characteristic (ROC) curve. To assess the best accuracy, the area under the curve (AUC) of the ROC was used to identify the optimal cut-off value for distinguishing two groups of samples. The optimum threshold was calculated "closest.topleft" method and 200 bootstrapping iterations in the pROC package in R. The method performed the formula is the minimal sum of $[(1\text{-sensitivity})^2 + (1\text{-specificity})^2]$. Logistic regression was used to estimate OR and 95% CI to describe the EC risk associated with the methylation status of each candidate gene and gene combination. All significant differences were assessed using a two-tailed P<0.05. Above analyses and plots were performed using the statistical package in R (version 3.1.2) and MedCalc (version 14.12.0). Pairwise Fisher's exact post hoc tests and an assumed alpha error proportion of 0.01 were used to evaluate the statistic power by G*Power version 3.1.9.2.

Example 1

**Identification of Hypermethylated Genes Associated with Diagnosis of Endometrial Cancer**

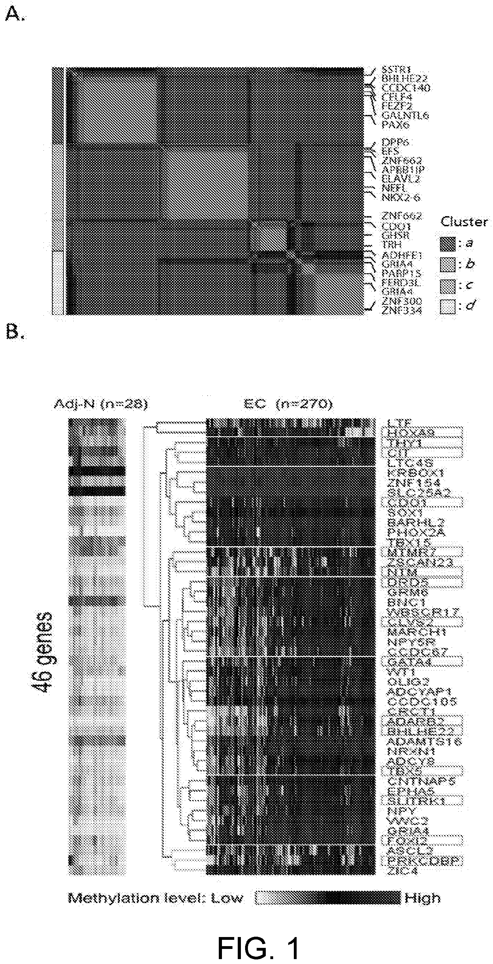

In the discovery cohort, novel hypermethylated genes in endometrial cancer were identified using data from MethyCap sequencing and methylation bead chip to identify DMRs that locate at $\pm 1$ kb spanning from the TSS of each gene. In the first analytic stage, the methylation levels of genes between endometrial carcinoma and normal samples from different datasets were compared, thereby identifying 180 genes with consensus clustering of methylation patterns. After filtering with our selection criteria for clinical relevance and functional enrichment, 23 genes are selected (see, panel A, Figure 1).

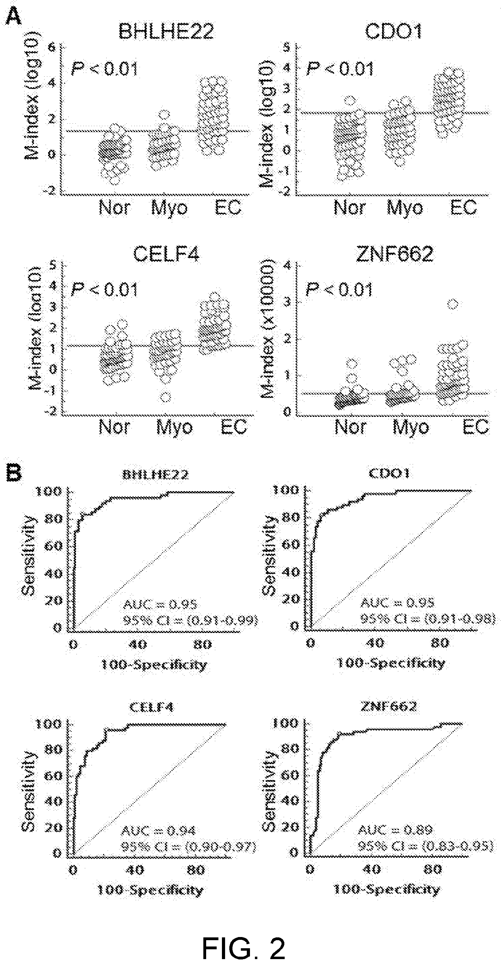

The 23 candidate genes identified in the discovery cohort were subjected to further verification in which pooled DNA from normal endometrial tissues (n = 15), myoma endometrium (n = 10), and malignant endometrial carcinoma tissue (n = 15) were subjected to qMSP, in which 14 gene were identified. The 14 candidate genes were subjected to further validation in which pooled DNA from cervical scrapings from patient diagnosed with malignant endometrial carcinoma (EC, n = 56) or myoma (Myo, n = 40) and from subjects with normal endometrial tissues (Nor, n = 50) were subjected to bisulfite pyrosequencing. The results (Figure 2 and Table 1) indicate that the above-mentioned nine genes are also capable of distinguishing subjects with endometrial carcinoma and normal or myoma endometrial tissue using cervical scrapings samples.

Table 1

| Gene Name | Cutoff value[a] | Positive number | | Se (%) | Sp (%) | OR[b] (95% CI) |
|---|---|---|---|---|---|---|
| | | case | control | | | |
| **Overall stage of EC** | | | | | | |
| BHLHE22 | 21.9 | 41/49 | 6/95 | 83.7 | 93.7 | 76.0 (24.8-233.3) |
| CDO1 | 67.5 | 41/50 | 6/96 | 82.0 | 93.8 | 68.3 (22.8-204.7) |
| CELF4 | 14.0 | 48/50 | 19/89 | 96.0 | 78.7 | 88.4 (19.7-397.3) |
| ZNF662 | 5034.8 | 46/50 | 19/96 | 92.0 | 80 | 46.6 (14.9-145.5) |
| BHLHE22 + CDO1 + CELF4 | any two Pos. | 45/49 | 4/88 | 91.8 | 95.5 | 236.3 (56.4-989.6) |
| **Early stage of EC** | | | | | | |

(continued)

| Early stage of EC | | | | | | |
|---|---|---|---|---|---|---|
| BHLHE22 + CDO1 + CELF4 | any two Pos. | 34/38 | 4/88 | 89.5 | 95.5 | 178.5 (42.2-754.9) |

Abbreviation: Se, sensitivity; Sp, specificity; OR, odds ratio; CI, confidence interval; EC, endometrial cancer. Pos., positive. The early stage includes stage I and stage II, [a]Dichotomization of methylation levels is based on the distribution in endometrial cancer vs. noncarcinomatous participants using Youden's method. [b]OR is calculated using univariate logistic regression. Actual $\alpha$ values ≤0.10 and powers $(1-\beta)$ >0.99 were calculated by post hoc pairwise Fisher's exact tests.

In particular, the dot plots in panel A of Figure 2 indicate the distribution of methylation status for BHLHE22, CDO1, CELF4, and ZNF662. Using individual cervical scrapings, it was confirmed that the DNA methylation status was higher in EC samples than in samples of normal endometrium and myoma. The horizontal line in the plot represented the cutoff values listed in Table 1. P values were calculated by the Kruskal-Wallis test.

Moreover, as demonstrated in panel B of Figure 2, all four genes exhibited an excellent area under the receiver-operating characteristic with their AUC value close to 1 (0.89 to 0.95), indicating a better cluster.

The performance of gene combination was also evaluated, and the results, as provided in Table 1, indicated that a better specificity was achieved using the gene combination, as compared with single gene. More specifically, the combined testing of BHLHE22, CELF4, and CDO1 had a sensitivity of 91.8%, specificity of 95.5%, and a 236.3-times elevated risk (95% CI, 56.4-989.6) for any two positive tests. These results were similar for the detection of EC at earlier stages (sensitivity, 89.5%; specificity, 95.5%; OR, 178.5, 95% CI, 42.2-754.9). The methylation status of candidate genes in cervical scrapings form some patients diagnosed with type II endometrioid cancer was provided in Table 2.

Table 2

| Participant No. | Age (years) | Histology | Stage | Grade | M-index of BHLHE22 | M-index of CELF4 | M-index of CDO1 | Any two Positive[a] |
|---|---|---|---|---|---|---|---|---|
| GYCA-03-2001 | 57 | Ser | 1A | G3 | *2579.2* | *577.1* | *1533.6* | yes |
| GYCA-03-2002 | 59 | En | 3C | G3 | 2.0 | *14.8* | *142.3* | yes |
| GYCA-03-2003 | 64 | Ser | 3C | G2 | *153.0* | *37.7* | *559.4* | yes |
| GYCA-03-2004 | 54 | En | 1B | G3 | *365.2* | 14.2 | *320.2* | yes |
| GYCA-03-2005 | 58 | En | 3A | G3 | *810.5* | *106.0* | *796.6* | yes |
| GYCA-03-2006 | 43 | En | 3A | G3 | *13707.8* | *563.3* | *3110.0* | yes |
| GYCA-03-2007 | 60 | Mu | 2 | G1 | 9.0 | *340.8* | *1353.7* | yes |
| GYCA-03-2008 | 73 | CC | 4B | G3 | *311.4* | *82.3* | *1725.4* | yes |
| GYCA-03-2009 | 74 | Ser | 1A | G3 | *37.9* | 6.4 | *1220.0* | yes |
| GYCA-03-2010[h] | 45 | Ser | 1 | | 5.9 | 10.4 | 6.4 | no |
| GYCA-03-2011 | 68 | Ser | 2 | G3 | *2044.8* | 10.8 | *2102.2* | yes |
| GYCA-03-2012 | 56 | Mu | 1B | G1 | *240.1* | *85.8* | *172.8* | yes |
| GYCA-03-2013 | 55 | Mu | 1A | G1 | *53.4* | *22.4* | 12.6 | yes |

(continued)

| Participant No. | Age (years) | Histology | Stage | Grade | M-index of *BHLHE22* | M-index of *CELF4* | M-index of *CDO1* | Any two Positive[a] |
|---|---|---|---|---|---|---|---|---|
| GYCA-03-2014 | 57 | Mu | 1A | G2 | ***375.5*** | ***172.2*** | ***217.2*** | yes |

Mu, mucinous; CG, clear cell; Ser, serous; En, endometrioid; G, grade; M-index, methylation index. Stage is following International Federation of Gynecology and Obstetrics princeipel. "The cutoff values based on the results calculated in endometrioid type. Cutoff values of M-index are 21.9, 14.0 and 69.5 for *BHLHE22, CELF4* and *CDO1,* respectively. The bold italics show that the M-index is higher than cutoff values. [b]The patient was diagnosed to be serous type of endometrial cancer by dilatation and curettage in outside clinic, but the hysterectomy specimens only disclosed complex atypical hyperplasia only in our hospital.

## Example 2

**Identification of Hypermethylated Genes Associated with Diagnosis of Endometrial Carcinoma**

In the discovery cohort, novel hypermethylated genes in endometrial cancer were identified using the datasets identified above. Differential methylation regions (DMRs), which locate at $\pm 1$ kb spanning from the transcription start site (TSS) of each gene were identified. In the first analytic stage, the methylation levels of genes between tumor and normal samples from the TCGA datasets were compared, thereby identifying 46 genes. Additionally, the methylation levels of genes between tumor and normal samples from the TCGA datasets were compared to identify candidate genes. A total of 15 genes that consistently exhibiting high methylation levels are identified (see, panel B, Figure 1).

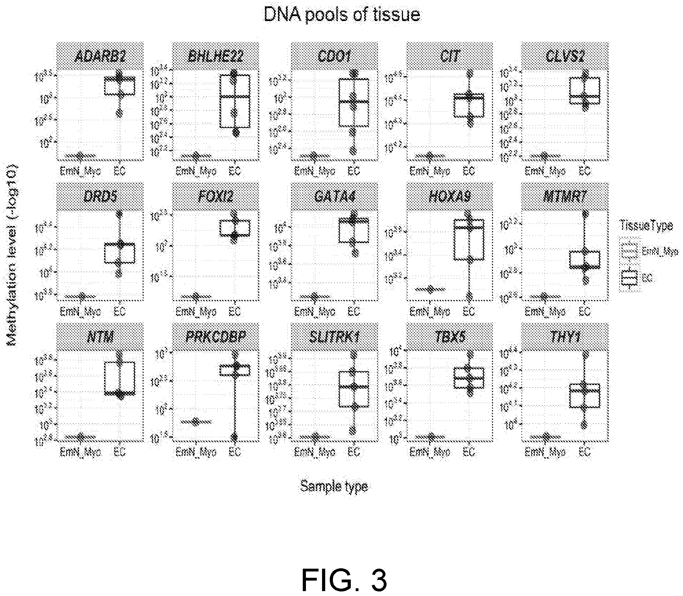

The 15 candidate genes identified in the discovery cohort were subjected to further verification in which pooled DNA from normal endometrium of myoma (EmN_Myo, n = 5) and malignant endometrial cancer tissues (EC, n = 25) were subjected to qMSP. The results (Figure 3) indicate that all 15 candidate genes exhibit significant hypermethylation in malignant endometrial cancer tissues.

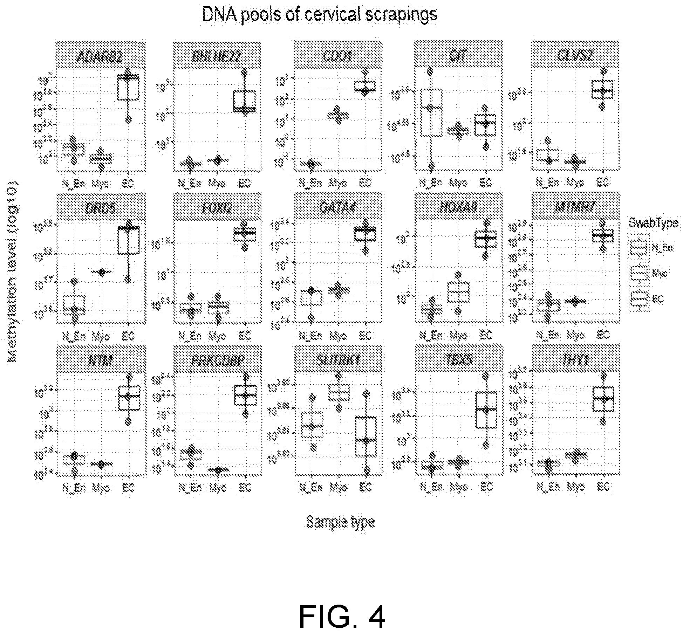

The candidate genes were also subject to further verification in which pooled DNA from cervical scrapings from subjects with normal endometrial tissues (N_Em, n = 15) or patient diagnosed with malignant endometrial tumor (EC, n = 15) or myoma (Myo, n = 10) were subjected to qMSP. The results (Figure 4) indicate that 11 out of the 15 genes are also capable of distinguishing subjects with malignant tumor and normal or myoma endometrial tissue using cervical scrapings samples. Said 11 genes are, ADARB2, BHLHE22, CDO1, CLVS2, GATA4, HOXA9, MTMR7, NTM, PRKCDBP, TBX5, and THY1. Note that although FOXI2 showed a differential methylation, the signal was relative weak, and hence, it was excluded from further investigation.

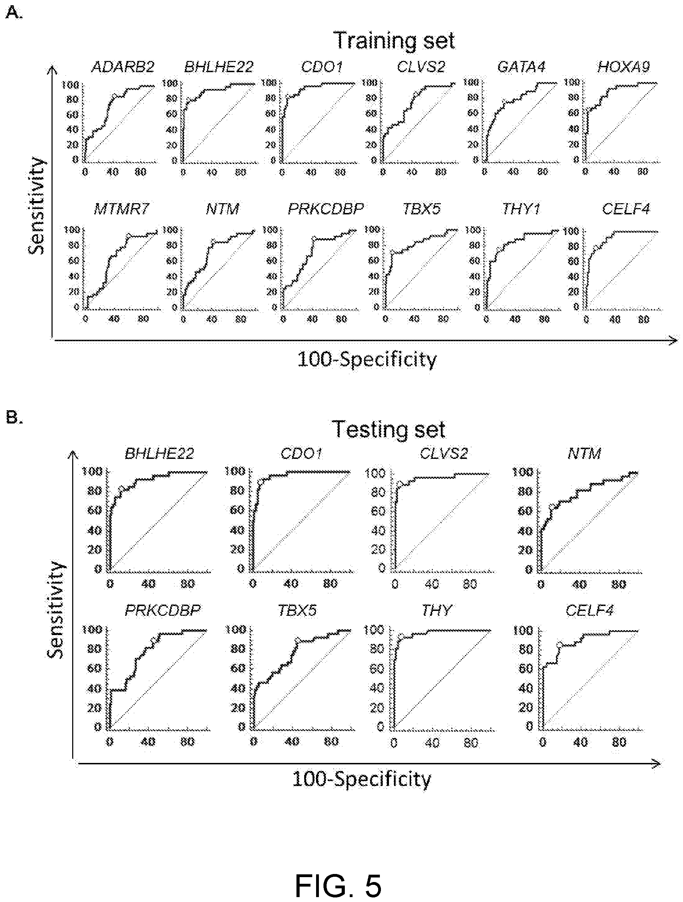

The methylation profiles of said eleven candidate genes, as well as a previously identified gene CELF4, were further investigated. Figure 5 demonstrates the performance of candidate genes for distinguishing of EC and non-EC samples using the area under the receiver-operating characteristic curve in training set (panel A) and testing set (panel B). P values for all the analyses were < 0.001 for comparison of the area equal 0.5. The threshold values were labeled with the open circles and listed in Table 3.

Table 3

| Gene Name | In training set | | | | In testing set | | | |
|---|---|---|---|---|---|---|---|---|
| | Threshold | Sen. | Spe. | AUC (95% CI) | Threshold | Sen. | Spe. | AUC (95% CI) |
| *ADARB2* | 6.37 | 78.57 | 63.64 | 0.75 (0.64 to 0.85) | | | | |
| *BHLHE22* | 8.83 | 73.57 | 92.86 | 0.91 (0.84 to 0.98) | 7.69 | 79.31 | 88.71 | 0.83 (0.90 to 0.97) |
| *CDO1* | 7.47 | 82.14 | 92.86 | 0.93 (0.88 to 0.99) | 6.97 | 89.66 | 88.71 | 0.89 (0.94 to 0.99) |
| *CELF4* | 8.42 | 73.57 | 35.71 | 0.91 (0.35 to 0.97) | 7.40 | 85.19 | 82.98 | 0.90 (0.81 to 0.96) |
| *CLVS2* | 6.75 | 67.86 | 67.86 | 0.76 (0.65 to 0.87) | 6.49 | 88.89 | 92.00 | 0.89 (0.95 to 1.0) |
| *GATA4* | 4.33 | 75.00 | 72.73 | 0.78 (0.67 to 0.88) | 4.69 | 74.07 | 82.00 | 0.68 (0.79 to 0.91) |
| *HOXA9* | 7.19 | 82.14 | 76.79 | 0.88 (0.81 to 0.96) | | | | |
| *MTMR7* | 4.99 | 67.86 | 64.29 | 0.66 (0.54 to 0.78) | | | | |
| *NTM* | 4.13 | 78.57 | 64.29 | 0.73 (0.52 to 0.85) | 3.80 | 68.97 | 79.03 | 0.69 (0.80 to 0.90) |
| *PRKCDBP* | 7.72 | 89.29 | 58.93 | 0.73 (0.52 to 0.85) | 5.87 | 68.97 | 69.35 | 0.65 (0.76 to 0.86) |
| *TBX5* | 3.81 | 71.43 | 91.07 | 0.83 (0.72 to 0.93) | 4.90 | 65.52 | 69.35 | 0.66 (0.77 to 0.88) |

(continued)

| Gene Name | In training set | | | | In testing set | | | |
|---|---|---|---|---|---|---|---|---|
| | Threshold | Sen. | Spe. | AUC (95% CI) | Threshold | Sen. | Spe. | AUC (95% CI) |
| THY1 | 2.35 | 75.00 | 83.64 | 0.85 (0.76 to 0.94) | 3.66 | 92.59 | 90.00 | 0.89 (0.95 to 0.99) |

Abbreviation: Sen., sencitivity; Sep., specificity, AUC, area under the curve of receiver operating characteristic curve; CI, conference interval. The ADARB2, HOXA9 and MTMR7 were unimproved the accuracy under the gene combination analysts in training set to exclude validation in the testing set.

The data in Table 3 indicates that six of the tested genes (BHLHE22, CDO1, CELF4, CLVS2, GATA4, and THY1) exhibited significant accuracies (at least 80%) in distinguishing subjects with the normal endometrium and myoma from those with endometrial carcinoma. Further, the AUC values of BHLHE22, CDO1, CELF4, CLVS2, and THY1 are in the range of 0.83 to 0.9, indicating a better cluster. Genes with the better accuracy were selected for subsequent analysis regarding the performance of gene combinations, and the results are summarized in Table 4.

Table 4

| Gene combination | Threshold of RS | Sen. | Spe. | AUC (95% CI) |
|---|---|---|---|---|
| BHLHE22 + CDO1 | -0.88 | 89.10 | 89.10 | 0.95 (0.90 to 0.98) |
| CDO1 + THY1 | -0.34 | 83.60 | 94.10 | 0.95 (0.90 to 0.98) |
| BHLHE22 + CDO1+TBX5 | -0.54 | 85.50 | 91.10 | 0.95 (0.90 to 0.98) |
| CDO1 + THY1 + CLVS2 | -0.07 | 83.64 | 94.10 | 0.95 (0.90 to 0.98) |
| CDO1 + THY1 + GATA4 | -1.17 | 90.91 | 85.15 | 0.95 (0.90 to 0.98) |
| CDO1 + THY1 + CLVS2 + GATA4 | -0.11 | 81.82 | 96.04 | 0.95 (0.90 to 0.98) |
| BHLHE22 + CDO1 + THY1 + CLVS2 | -0.49 | 87.27 | 92.08 | 0.95 (0.90 to 0.98) |
| 9 genes: BHLHE22 + CDO1 + THY1 + CLVS2 + GATA4 + NTM + PRKCDBP+TBX5+CELF4 | -0.18 | 95.45 | 96.04 | 0.96 (0.91 to 0.98) |

Abbreviation: RS, risk score; Sen., sencitivity; Sep., specificity, AUC, area under the curve of receiver operating characteristic curve: CI, conference interval. The risk score was calculated by using total cervical scrapings and logistic regression model.

The data in Table 4 demonstrate that these gene combination has a better accuracy than any single gene by using total cervical scrapings (Table 4, 56 EC, 56 myoma, and 56 normal).

## EXAMPLE 3

## CROSSED TEST CANDIATE GENES IN OTHER GYNECOLOGICAL CARCINOMA AND COLON CARCINOMA

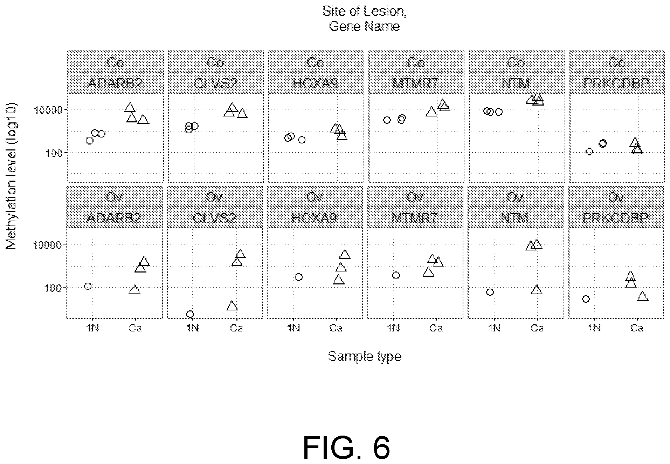

ADARB2, CLVS2, HOXA9, MTMR7, NTM, and PRKCDBP were further investigated for their ability in distinguishing abnormal gynecologic diseases or colon carcinoma, and the results are summarized in Figure 6 (each symbol represents the data from the pooled DNA of 5 people). In colon tissue samples (Co), ADARB2, CLVS2, HOXA9, MTMR7 and NTM exhibited the high methylation in colon cancers (Ca), as compared with the methylation level in normal colon tissue (1N). In ovarian (Ov) tissues, all seven genes exhibited a high methylation level in ovarian cancers. Each triangle and circle are pooled by 5 equal amounts of DNA of samples.

It will be understood that the above description of embodiments is given by way of example only and that various modifications may be made by those with ordinary skill in the art. The above specification, examples, and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

**Claims**

1. An *ex vivo* method for assessing whether a subject has an endometrial carcinoma, the method comprising determining a methylation state of a target gene as a biomarker in a sample derived from the endometrial tissue or cervical scraping cells of the subject, wherein the target gene is BHLHE22 or THY1; and the hypermethylation of the biomarker is an indication that the subject has an endometrial carcinoma.

2. The method according to claim 1, wherein the target gene further comprises CDO1.

3. The method according to claim 1, wherein the target gene further comprises at least one of ADARB2, CDO1, CELF4, CLVS2, GATA4, HOXA9, MTMR7, NTM, PRKCDBP, TBX5, and ZNF662.

4. The method according to claim 3, wherein the target gene comprises BHLHE22, CDO1, THY1, CELF4, CLVS2, GATA4, NTM, PRKCDBP, and TBX5.

5. The method according to claim1, wherein the sample is derived from cervical scraping cells of the subject, the biomarker comprises BHLHE22 and at least one of CDO1 and CELF4.

6. The method according to claim 1, wherein the endometrial carcinoma is type I endometrial carcinoma or type II endometrial carcinoma.

**Patentansprüche**

1. Ex vivo-Verfahren zum Abschätzen, ob ein Proband ein Endometriumkarzinom aufweist, wobei das Verfahren Bestimmen eines Methylierungszustandes eines Zielgens als einen Biomarker in einer Probe, abgeleitet von dem Endometriumgewebe oder Gebärmutterhals-Abschabungszellen von dem Probanden, umfasst, wobei das Zielgen BHLHE22 oder THY1 ist; und die Hypermethylierung des Biomarkers ein Anzeichen, dass der Proband ein Endometriumkarzinom aufweist, ist.

2. Verfahren gemäß Anspruch 1, wobei das Zielgen ferner CDO1 umfasst.

3. Verfahren gemäß Anspruch 1, wobei das Zielgen ferner mindestens eines von ADARB2, CDO1, CELF4, CLVS2, GATA4, HOXA9, MTMR7, NTM, PRKCDBP, TBX5, und ZNF662 umfasst.

4. Verfahren gemäß Anspruch 3, wobei das Zielgen BHLHE22, CDO1, THY1, CELF4, CLVS2, GATA4, NTM, PRKCDBP, und TBX5 umfasst.

5. Verfahren gemäß Anspruch 1, wobei die Probe von Gebärmutterhals-Abschabungszellen von dem Probanden abgeleitet ist, wobei der Biomarker BHLHE22 und mindestens eines von CDO1 und CELF4 umfasst.

6. Verfahren gemäß Anspruch 1, wobei das Endometriumkarzinom Typ I-Endometriumkarzinom oder Typ 11-Endometriumkarzinom ist.

**Revendications**

1. Procédé ex *vivo* permettant d'évaluer si un sujet est atteint d'un carcinome de l'endomètre, le procédé comprenant la détermination d'un état de méthylation d'un gène cible en tant que biomarqueur dans un échantillon issu du tissu endométrial ou de cellules de curetage du col de l'utérus du sujet, dans lequel le gène cible est BHLHE22 ou THY1 ; et l'hyperméthylation du biomarqueur est une indication que le sujet est atteint d'un carcinome de l'endomètre.

2. Procédé selon la revendication 1, dans lequel le gène cible comprend en outre CDO1.

**3.** Procédé selon la revendication 1, dans lequel le gène cible comprend en outre au moins un parmi ADARB2, CDO1, CELF4, CLVS2, GATA4, HOXA9, MTMR7, NTM, PRKCDBP, TBX5 et ZNF662.

**4.** Procédé selon la revendication 3, dans lequel le gène cible comprend BHLHE22, CDO1, THY1, CELF4, CLVS2, GATA4, NTM, PRKCDBP, et TBX5.

**5.** Procédé selon la revendication 1, dans lequel l'échantillon est issu de cellules de curetage du col de l'utérus du sujet, le biomarqueur comprend BHLHE22 et au moins un parmi CDO1 et CELF4.

**6.** Procédé selon la revendication 1, dans lequel le carcinome de l'endomètre est un carcinome de l'endomètre de type I ou un carcinome de l'endomètre de type II.

A.

B.

FIG. 1

FIG. 2

DNA pools of tissue

FIG. 3

DNA pools of cervical scrapings

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHEN, Y.C. et al.** *TAIWANESE JOURNAL OF OBSTETRICS & GYNECOLOGY,* 31 December 2015, vol. 54 (5), 572-579

- **YOON, M.S. et al.** *ONCOLOGY REPORTS,* 31 December 2010, 917-925